# FASCICULE DE BREVET EUROPEEN

(11) **EP 1 661 551 B1**
(45) Date de publication et mention de la délivrance du brevet: **20.08.2014**
(21) Numéro de dépôt: 05292406.5
(22) Date de dépôt: 14.11.2005
(51) Int. Cl.: A61K 8/42, A61Q 5/04

(54) **Procédé de défrisage des fibres kératiniques avec un moyen de chauffage et un agent dénaturant**
Verfahren zum Glätten von Keratinfasern durch eine Heizvorrichtung und ein Denaturierungsmittel
Method of straightening keratin fibres with heating means and a denaturant agent

(30) Priorité: 26.11.2004 FR 0452775
(43) Date de publication de la demande: 31.05.2006
(73) Titulaire: L'Oréal, 75008 Paris (FR)
(72) Inventeur: Radisson, Xavier, 92600 Asnières sur Seine (FR); Malle, Gerard, 77580 Villiers s/Morin (FR); Barbarat, Philippe, 92270 Bois-Colombes (FR); Diridollou, Stephane, 60657 Chicago (US)
(74) Mandataire: Kromer, Christophe

(56) Documents cités:
- EP-A- 0 190 834
- EP-A- 0 658 338
- EP-A- 1 468 667
- EP-A- 1 532 963
- EP-A- 1 535 598
- WO-A-01/47482
- WO-A-2004/002263
- FR-A- 2 829 925
- GB-A- 1 076 420
- GB-A- 1 144 308
- GB-A- 1 466 622
- JP-A- 2003 212 737
- PATENT ABSTRACTS OF JAPAN vol. 2003, no. 12, 5 décembre 2003 (2003-12-05) -& JP 2004 149478 A (HOYU CO LTD), 27 mai 2004 (2004-05-27) & JP 2004 149478 A (HOYU CO LTD) 27 mai 2004 (2004-05-27)
- DATABASE WPI Section Ch, Week 200414 Derwent Publications Ltd., London, GB; Class A96, AN 2004-137526 XP002339007 & JP 2004 002459 A (MIRUBON KK) 8 janvier 2004 (2004-01-08)
- DATABASE CAPLUS [Online] CHEMICAL ABSTRACTS SERVICE, COLUMBUS, OHIO, US; KOBAYASHI, MIKIO ET AL.: "hair straightening or wave-setting compositions containing protein denaturants" XP002338918 extrait de CAPLUS Database accession no. 2003:585180 & JP 2003 212737 A (HOYU CO LTD) 30 juillet 2003 (2003-07-30)
- PATENT ABSTRACTS OF JAPAN vol. 2003, no. 04, 2 avril 2003 (2003-04-02) -& JP 2002 356408 A (MILBON CO LTD), 13 décembre 2002 (2002-12-13)
- DATABASE WPI Section Ch, Week 199703 Derwent Publications Ltd., London, GB; Class A96, AN 1997-029432 XP002394770 & JP 08 291029 A (HOYU KK) 5 novembre 1996 (1996-11-05)
- PATENT ABSTRACTS OF JAPAN vol. 1995, no. 08, 29 septembre 1995 (1995-09-29) & JP 07 138134 A (SHISEIDO CO LTD), 30 mai 1995 (1995-05-30)

## Description

L'invention a pour objet un procédé de défrisage des fibres kératiniques avec un moyen de chauffage et au moins un agent dénaturant.

Par *"fibres kératiniques",* on entend, des fibres d'origine humaine ou animale telles que les cheveux, les poils, les cils, la laine, l'angora, le cachemire ou la fourrure. L'invention fait référence aux cheveux.

Le terme *"défrisage"* englobe, selon l'invention, le défrisage, de cheveux caucasiens ou africains. Le terme *« défriser »* englobe, selon l'invention, défriser, des cheveux caucasiens ou africains.

On entend par *« argent dénaturant »,* un composé organique ou minéral possédant à la fois au moins un site donneur d'électrons à caractère basique ou nucléophile et au moins un site accepteur d'électrons à caractère acide ou électrophile, interagissant avec les liaisons faibles de la kératine.

Selon l'invention, un agent dénaturant est un composé capable de diminuer le pouvoir rotatoire d'une protéine modèle telle que par exemple la bovine serum albumine d'au moins 7° et/ou 5° à 579nm, les mesures étant effectuées après 3h d'incubation à 37°C, à l'aide d'un polarimètre, tel que décrit dans Biochemistry 2 (1), 47-57, 1963 :
- soit dans un tampon TRIS 0.05M pH 7,6
- soit dans une solution d'urée 5,45M lorsque la solubilité du composé est insuffisante dans le tampon TRIS 0.05M pH 7,6.

Le composé est considéré comme agent dénaturant selon l'invention si la diminution du pouvoir rotatoire est d'au moins 7° dans le tampon TRIS 0.05M pH 7,6 et/ou d'au moins 5° dans la solution d'urée 5,45M.

Par *« liaisons faibles de la kératine »,* on entend l'ensemble des liaisons non covalentes telles que :
- les liaisons salines résultant d'interactions coulombiennes entre les groupes fonctionnels présents au niveau des chaînes latérales des acides aminés
- les liaisons hydrogènes qui s'établissent entre les acides aminés par l'intermédiaire notamment des atomes d'oxygène et d'hydrogène
- les liaisons hydrophobes qui résultent de la tendance des chaînes non polaires des acides aminés à s'associer pour minimiser les contacts avec l'eau

Par «*moyen de chauffage»,* on entend tout moyen permettant de chauffer les fibres kératiniques à une température d'au moins 110°C tels que les fers chauffants, par \ exemple les fers plats ou ronds, les générateurs de micro-ondes, les sources de rayonnement infra-rouge.

Deux techniques sont utilisées pour obtenir une déformation permanente des cheveux. Elles sont basées sur une rupture des liaisons covalentes disulfures présentes dans la kératine (cystine) :
- la première consiste, dans un premier temps à réaliser cette ouverture des liaisons disulfures à l'aide d'une composition contenant un agent réducteur, puis après avoir, de préférence, rincé les cheveux, à reconstituer dans un second temps les dites liaisons disulfures en appliquant sur les cheveux préalablement mis sous tension par des bigoudis ou autres ou mis en forme ou lissés par d'autres moyens, une composition oxydante encore appelée fixateur, de façon à donner à la chevelure la forme recherchée. Cette technique permet indifféremment de réaliser, soit l'ondulation des cheveux, soit leur défrisage ou leur décrêpage ou leur lissage.
- La seconde consiste à effectuer une opération dite de lanthionisation, à l'aide d'une composition contenant une base appartenant à la famille des hydroxydes. Elle conduit à remplacer des liaisons disulfures (-CH2-S-S-CH2-) par des liaisons lanthionines (-CH2-S-CH2-). Cette opération de lanthionisation fait intervenir deux réactions chimiques consécutives :
   ■ La première réaction consiste en une béta-élimination sur la cystine provoquée par un ion hydroxyde, conduisant à la rupture de cette liaison et à la formation de déhydro-alanine. 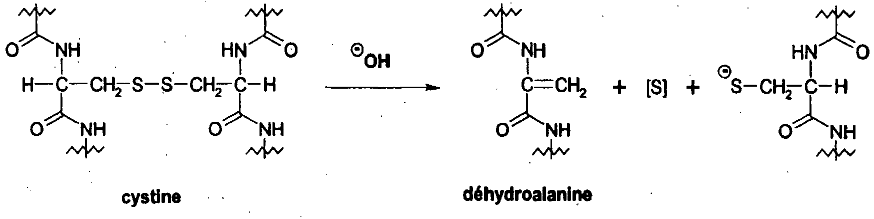
   ■ La deuxième réaction est une réaction de la déhydro-alanine avec un groupe thiol. En effet, la double-liaison de la déhydro-alanine formée est une double-liaison réactive. Elle peut réagir avec le groupe thiol du résidu cystéine qui a été libéré pour former une nouvelle liaison appelée pont ou liaison ou résidu lanthionine.

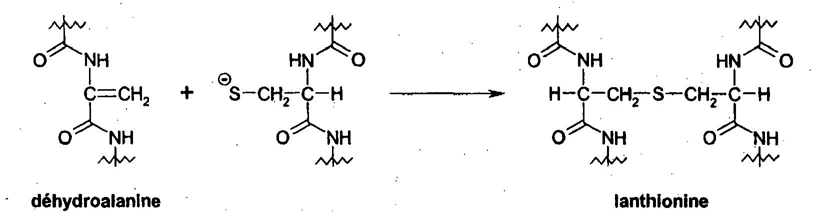

Par rapport à la première technique mettant en oeuvre un agent réducteur, cette technique de lanthionisation ne nécessite pas d'étape de fixation, puisque la formation des ponts lanthionine est irréversible. Elle s'effectue donc en une seule étape et permet indifféremment de réaliser soit l'ondulation des cheveux, soit leur défrisage ou leur décrêpage ou leur lissage. Cependant, elle est principalement utilisée pour le défrisage des cheveux naturellement crépus.

Pour la première technique, les compositions réductrices généralement utilisées pour la première étape d'une opération de permanente ou de défrisage contiennent à titre d'agent réducteur des thiols ou des sulfites ou des bisulfites. Ces agents sont généralement employés en milieu essentiellement aqueux à des concentrations comprises entre 0,5 et 1M pour obtenir une bonne ouverture des liaisons disulfures. Parmi les thiols, ceux couramment utilisés sont l'acide thioglycolique, la cystéamine, le monothioglycolate de glycérol, l'acide thiolactique et la cystéine. L'acide thioglycolique est particulièrement efficace pour réduire les liaisons disulfures de la kératine à pH alcalin, notamment sous forme de thioglycolate d'ammonium, et constitue le produit le plus utilisé en permanente (" hair waving "). On a toutefois constaté que l'acide thioglycolique doit être utilisé en milieu suffisamment basique (en pratique à pH compris entre 8,5 et 9,5) si on veut obtenir une frisure satisfaisante en intensité. Outre l'inconvénient de dégager une odeur désagréable nécessitant l'usage de parfums plus ou moins efficaces pour masquer les odeurs, l'utilisation d'un thiol à pH alcalin conduit également à des dégradations de la fibre et tout particulièrement à l'altération des colorations artificielles.

Les sulfites ou bisulfites sont principalement utilisés pour le défrisage. Ils possèdent des désavantages similaires aux thiols avec une efficacité moindre.

Les thiols et les sulfites (ou bisulfites) présentent en outre l'inconvénient d'avoir une mauvaise stabilité en solution aqueuse.

D'une façon générale, la durabilité des effets de déformations obtenus avec les thiols et les sulfites par réduction des disulfures puis fixation est jugée très inférieure à celle pouvant être obtenue par la technique de lanthionisation.

Pour la deuxième technique, les compositions généralement utilisées pour effectuer la lanthionisation contiennent à titre de base un hydroxyde tel que l'hydroxyde de sodium, l'hydroxyde de guanidinium et l'hydroxyde de lithium. Ces actifs de lanthionisation, permettant d'ouvrir les liaisons disulfures par un mécanisme de béta-élimination, sont généralement employés en émulsion eau-huile à des concentrations comprises entre 0,4 et 0,6M, en les laissant agir généralement 10 à 15 minutes à température ambiante. L'hydroxyde de sodium reste l'agent le plus utilisé. L'hydroxyde de guanidinium est maintenant le composé préféré pour de nombreuses compositions. Ces deux hydroxydes, de sodium et de guanidinium, sont les deux agents principaux utilisés pour le défrisage ou le décrêpage des cheveux naturellement crépus. Ils possèdent plusieurs avantages par rapport au thioglycolate d'ammonium et aux sulfites, en particulier une absence d'odeur désagréable, le fait qu'une seule étape de mise en oeuvre est requise (durée du traitement plus courte), et une durabilité et une efficacité beaucoup plus importante de la déformation du cheveu.
Cependant, ces hydroxydes présentent l'inconvénient majeur d'être caustiques. Cette causticité affecte le cuir chevelu en provoquant des irritations parfois sévères. On peut y remédier partiellement par l'application préalable sur le scalp d'une crème protectrice grasse souvent appelée " base " ou " crème base ", le mot " base " ici utilisé n'ayant pas la signification d'agent basique au sens chimique. Lorsque la crème protectrice est associée à l'hydroxyde en une seule composition, celle-ci est généralement appelée " no-base", par opposition à l'appellation ci-dessus. Cette technologie " no-base " est préférée.

La causticité des hydroxydes affecte également l'état du cheveu en le rendant d'une part rêche au toucher et d'autre part beaucoup plus fragile, cette fragilité pouvant aller jusqu'à l'effritement voire la rupture ou même la dissolution des cheveux si le traitement est prolongé. Les hydroxydes provoquent dans certains cas également des décolorations de la couleur naturelle du cheveu.

Les formules contenant de l'hydroxyde de sodium sont généralement dénommées en anglais " lye relaxers" et celles n'en contenant pas sont dénommées " no-lye relaxers ".

Les principales formules défrisantes dites " no-lye " mettent en oeuvre l'hydroxyde de guanidinium. L'hydroxyde de guanidinium étant instable, celui-ci est généré extemporanément par le mélange de carbonate de guanidine et d'une source d'hydroxyde très peu soluble telle que l'hydroxyde de calcium. La réaction entre ces deux composés conduit à la formation d'hydroxyde de guanidinium et de carbonate de calcium qui précipite dans la composition. La présence de ce précipité rend le rinçage final des cheveux beaucoup plus difficile et laisse sur les cheveux et le scalp des particules minérales qui lui donnent un toucher rêche et une apparence inesthétique ressemblant aux pellicules. Le succès récent de l'hydroxyde de guanidinium (" no-lye ") face à l'hydroxyde de sodium (" lye ") semble provenir d'une meilleure efficacité de défrisage et d'une meilleure tolérance cutanée. Cependant ces technologies mettant en oeuvre des bases de la famille des hydroxydes demeurent très agressives pour le cheveu et le cuir chevelu et demandent un contrôle très strict de la durée d'application pour éviter les irritations trop fortes et l'altération des cheveux pouvant aller jusqu'à la cassure. Cette agressivité provenant de la causticité des hydroxydes justifie que ces compositions pour la lanthionisation du cheveu ne soient pas utilisées pour la permanente (" hair waving ") mais réservées au défrisage (" hair straightening " ou " hair relaxing ").

De plus, les hydroxydes sont connus pour être de bons agents d'hydrolyse des fonctions amides (cf par exemple March's Advanced Organic Chemistry, 5ed., Wiley Interscience, New York, "Hydrolysis of Amides " pages 474 et suivantes) qui conduisent donc à la rupture des liaisons peptidiques par attaque nucléophile directe. Il est ainsi probable que les altérations constatées au niveau des cheveux et des matières kératiniques au sens large soient en grande partie dues à une hydrolyse partielle des liaisons amides de la kératine.

Il existe donc un réel besoin pour le défrisage de compositions nettement moins agressives pour le cheveu.

Diverses études ont été conduites en vue de remédier à la fois aux inconvénients des agents réducteurs (première technique) et/ou des hydroxydes (deuxième technique).

Ainsi pour remplacer l'acide thioglycolique, de nombreux agents réducteurs ont été proposés, mais l'acide thioglycolique sous sa forme de thioglycolate d'ammonium reste à la fois le composé de référence et le plus largement utilisé dans les formulations cosmétiques, aussi bien pour la mise en forme que pour le lissage.

Il a également été proposé dans de très nombreux brevets d'associer des agents réducteurs habituels (thiols ou sulfites ou bisulfites) avec de l'urée ou des alkylurées pour diminuer l'irritation et les dommages causés aux cheveux aussi bien pour la mise en forme que pour le défrisage. On citera par exemple :
- la demande CA 1315204 qui décrit une composition contenant du thioglycolate d'ammonium (5,5-11,5%) et de l'urée ou une monoalkylurée (1-3%) pour la mise en forme des cheveux,
- la demande US 3847165 qui décrit une composition contenant du thioglycolate d'ammonium (1,2-1,4M) et de l'urée (2,0-2,7M) pour la mise en forme des cheveux à un pH acide,
- la demande NL 6410355 qui décrit une composition contenant un sulfite (0,8-1,5M) et de l'urée (0,6-3,0M) pour la mise en forme et le défrisage des cheveux,
- la demande JP 2000/229819 qui décrit une composition contenant un sulfite ou bisulfite (0,5-15%), de l'urée (0,5-15%) et un alcool (éthanol et/ou isopropanol, 1-30%) pour la mise en forme et le défrisage des cheveux,

Il a également été proposé dans de très nombreux brevets d'associer des hydroxydes, servant d'actif de lanthionisation, avec certains additifs servant généralement à protéger les cheveux. On citera, à titre d'exemple:
- la demande WO2002/003937, qui décrit une composition contenant des monosaccharides en C3-C5,
- la demande WO2001/064171, qui décrit une composition contenant des agents complexants,
- le brevet US5641477, qui décrit une composition contenant un hydrolysat d'amidon hydrogéné,
- la demande WO02085317, qui décrit une composition contenant des nucléophiles organiques qui réagissent lors de la deuxième étape avec la déhydro-alanine formée avec des hydroxydes, pour conduire à de nouveaux pontages.

Si toutes ces propositions conduisent à des améliorations plus ou moins marquées, elles ne permettent pas de diminuer de façon suffisante les dégâts liés à la causticité même des hydroxydes.

Comme indiqué précédemment, l'utilisation d'agents réducteurs conduit à une durabilité médiocre pour le défrisage ou le décrêpage et l'emploi d'hydroxydes, de part leur causticité, limite leur utilisation au domaine du défrisage.

Après d'importantes études, on vient maintenant de découvrir, de façon tout à fait surprenante et inattendue, que l'on pouvait défriser durablement le cheveu en associant l'action d'un dénaturant et d'un moyen de chauffage à une température supérieure à 110°C. On obtient ainsi d'excellents résultats en terme de défrisage, de propriétés cosmétiques des cheveux et d'intégrité de la fibre.

Sans être liée par la théorie, la Demanderesse pense qu'il existe une action conjointe, sur les fibres kératiniques, d'un agent dénaturant et d'un moyen de chauffage, qui permet de les défriser de façon efficace et durable.

La Demanderesse a trouvé qu'il était possible de remédier aux inconvénients de l'art antérieur et de remplir les objectifs précités en mettant en oeuvre un procédé de défrisage des fibres kératiniques comprenant :
- une étape d'application sur les fibres kératiniques d'une composition de défrisage contenant au moins un agent dénaturant de masse moléculaire supérieure à 18,1 g/mol, présent à une concentration molaire comprise entre 1M et 8M,
- une étape d'élévation de la température des fibres kératiniques, à l'aide d'un moyen de chauffage, à une température comprise entre 110 et 250°C, l'agent dénaturant étant une urée.

Une concentration molaire comprise entre 1M et 8M correspond à une concentration pondérale comprise entre environ 6 % et environ 80 % par rapport au poids total de la composition.

Ainsi, l'invention a pour objet un procédé de défrisage des cheveux comprenant :
- (i) une étape d'application sur les cheveux d'une composition de défrisage contenant au moins un agent dénaturant de masse moléculaire supérieure à 18,1 g/mol, et étant une urée de formule (I) 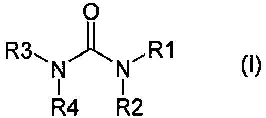 dans laquelle :
      R1, R2, R3, R4 représentent, indépendamment: un atome d'hydrogène ou un radical alkyle ou alcényl inférieur en C1-C4, linéaire ou ramifié, éventuellement substitué par un radical choisi parmi: hydroxyle, amino, diméthylamino, carboxyle ou carboxamide ou N-méthylcarboxamide
         - lorsque R1, R2 et R3 représentent un atome d'hydrogène, R4 peut également désigner un radical carboxamide; méthoxy; éthoxy; 1,2,4-triazolyl; cyclopentyl; méthoxycarbonyle; éthoxycarbonyle; CO-CH=CH-COOH; phényle éventuellement sustitué par un atome de chlore ou un radical hydroxyle; benzyle; ou 2,5-dioxo-4-imidazolidinyle
         - lorsque R1 et R3 représentent un atome d'hydrogène, R2 peut également représenter un atome d'hydrogène ou un radical méthyle ou éthyle et R4 un radical acétyle.
         - lorsque R1=R2=H, R3 et R4 peuvent également former, avec l'atome d'azote qui les porte, un cycle pipéridine ou 3-méthylpyrazole ou 3,5-diméthylpyrazole ou maléimide.
         - R1 et R2 ainsi que R3 et R4 peuvent également former, avec l'atome d'azote qui les porte, un cycle imidazole, l'agent dénaturant étant présent à une concentration molaire comprise entre 2M et 8M,
   (ii) une étape d'élévation de la température des cheveux, à l'aide d'un moyen de chauffage, à une température comprise entre 110 et 250°C,après l'étape d'application de la composition de défrisage et avant l'étape d'élévation de la température, on laisse poser la composition pendant 5 à 45 minutes ;
      l'agent dénaturant urée de formule (I) étant le seul actif de défrisage.

La composition de défrisage comprend entre 2M et 8M dudit agent dénaturant ; ce qui correspond à une concentration pondérale comprise entre environ 12% et environ 80%, par rapport au poids total de la composition, dudit agent dénaturant.

Avantageusement, on élève la température à l'aide du moyen de chauffage à une température comprise entre 120°C et 220°C, plus avantageusement entre 140°C et 220°C.

Avantageusement, la masse molaire du dénaturant est comprise entre 40 et 600 g/mol.

De préférence, ladite composition est appliquée sur des fibres kératiniques humides.

On peut également avantageusement intercaler entre l'étape d'application de la composition et l'étape d'élévation de température, une étape destinée à éliminer l'excédant de la composition, par exemple au moyen d'une serviette.

Par "urée", utilisable à titre d'actif de défrisage, on entend tout dérivé comprenant dans sa formule chimique un groupe carbonyle simplement lié à 2 atomes d'azote. Ces urées sont plus particulièrement sélectionnées parmi les composés de formules générales (I) (ci-dessous : 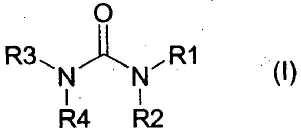 dans laquelle :
R1, R2, R3, R4 représentent, indépendamment :
   (i) un atome d'hydrogène ou
   (ii) un radical alkyle ou alcényl inférieur en C1-C4, linéaire ou ramifié, éventuellement substitué par un radical choisi parmi : hydroxyle, amino, diméthylamino, carboxyle ou carboxamide ou N-méthylcarboxamide

Lorsque R1, R2 et R3 représentent un atome d'hydrogène, R4 peut également désigner un radical carboxamide ; méthoxy ; éthoxy ; 1,2,4-triazolyl ; cyclopentyl ; méthoxycarbonyle; éthoxycxrbonyle ; CO-CH=H-COOH ; phényle éventuellement sustitué par un atome de chlore ou un radical hydroxyle ; benzyle ; ou 2,5-dioxo-4-imidazolidinyle

Lorsque R1 et R3 représentent un atome d'hydrogène, R2 peut également représenter un atome d'hydrogène ou un radical méthyle ou éthyle et R4 un radical acétyle.

Lorsque R1=R2=H, R3 et R4 peuvent également former, avec l'atome d'azote qui les porte, un cycle pipéridine ou 3-méthylpyrazole ou 3,5-diméthylpyrazole ou maléimide.

Enfin, R1 et R2 ainsi que R3 et R4 peuvent également former, avec l'atome d'azote qui les porte, un cycle imidazole.

Parmi les composés de formule (I), on peut notamment citer les composés préférés suivants :
- l'urée
- la méthylurée
- l'éthylurée
- la propylurée
- l'isopropylurée
- la n-butylurée
- la sec-butylurée
- l'isobutylurée
- la tert-butylurée
- la cyclopentylurée
- la 1-éthoxyurée
- la 2-hydroxyéthylurée
- la N-(2-hydroxypropyl)urée
- la N-(3-hydroxypropyl)urée
- la N-(2-diméthylaminopropyl)urée
- la N-(3-diméthylaminopropyl)urée
- la 1-(3-hydroxyphényl)urée
- la benzylurée
- le N-carbamoylmaléimide
- le biuret
- l'acide N-carbamoyl maléamique
- le 1-pipéridinecarboxamide
- la 1,2,4-triazol-4-yl-urée
- l'acide hydantoïque
- l'allophanate de méthyle l'allophanate d'éthyle
- l'acétylurée
- la 2-hydroxyéthylèneurée
- la 2-(hydroxyéthyl)éthylèneurée
- la N-allyl-N'-éthylurée
- la diallylurée
- la 2-chloroéthylurée
- la N,N-diméthylurée
- la N,N-diéthylurée
- la N,N-dipropylurée
- la 1-cyclopentyl-1-méthylurée
- la 1,3-diméthylurée
- la 1,3-diéthylurée
- la 1,3-bis(2-hydroxyéthyl)urée
- la 1,3-bis(2-hydroxypropyl)urée
- la 1,3-bis(3-hydroxypropyl)urée
- la 1,3-dipropylurée
- la 1-éthyl-3-propylurée
- la 1-sec-butyl-3-méthylylurée
- la 1-isobutyl-3-méthylurée
- la 1-cyclopentyl-3-méthylurée
- la N-acétyl-N'-méthylurée
- la triméthylurée
- la 1-butyl-3,3-diméthylurée
- la tétraméthylurée
- la benzylurée

Parmi les composés de formule (I), on peut notamment citer les composés particulièrement préférés suivants :
- l'urée
- la méthylurée
- l'éthylurée
- la propylurée
- la 1-éthoxyurée
- la 2-hydroxyéthylurée
- la N-(2-hydroxypropyl)urée
- la N-(3-hydroxypropyl)urée
- la N-(2-diméthylaminopropyl)urée
- la N-(3-diméthylaminopropyl)urée
- la 1-(3-hydroxyphényl)urée
- le N-carbamoylmaléimide
- l'acide N-carbamoyl maléamique
- le 1-pipéridinecarboxamide
- la 1,2,4-triazol-4-yl-urée
- l'acide hydantoïque
- l'acétylurée
- la 2-hydroxyéthylèneurée
- la 2-(hydroxyéthyl)éthylèneurée
- la N-allyl-N'-éthylurée
- la diallylurée
- la 2-chloroéthylurée
- la N,N-diméthylurée
- la 1,3-diméthylurée
- la 1,3-diéthylurée
- la 1,3-bis(2-hydroxyéthyl)urée
- la 1,3-dipropylurée
- la 1-éthyl-3-propylurée
- la N-acétyl-N'-méthylurée
- la benzylurée

Dans les compositions selon l'invention destinées à un processus de défrisage, des cheveux, l'urée de formule (I) est présente à une concentration molaire comprise entre 2M et 8M.

Le pH des compositions selon l'invention est, de préférence, compris entre 3 et 10, et plus particulièrement compris entre 5 et 9.

Dans les compositions de l'invention, l'urée de formule (I) constitue le seul actif de défrisage.

Les compositions selon l'invention se présentent soit sous la forme d'une solution aqueuse, soit sous forme d'une crème épaissie de façon à maintenir les cheveux aussi raides que possible. On réalisé ces crèmes, sous forme d'émulsions « lourdes ».

Dans le but d'améliorer les propriétés cosmétiques des fibres kératiniques ou encore d'atténuer ou d'éviter leur dégradation, la composition utilisée selon l'invention peut également comprendre un ou plusieurs actifs cosmétiques supplémentaires.

Généralement, le ou lesdits actifs cosmétiques supplémentaires représentent de 0,01 à 30%, de préférence de 0,1 à 10%, en poids du poids total de la composition cosmétique.

Généralement, la composition appliquée sur les fibres kératiniques est appliquée à hauteur de 0,05 à 20 g, de préférence de 0,1 à 10 g de composition par gramme de fibre kératinique sèche.

Après application de la composition, et avant l'élévation de la température des fibres kératiniques au moyen d'un moyen de chauffage, on laisse poser ladite composition, pendant 5 à 45 minutes.

Le procédé selon l'invention comprend, après l'étape d'application de la composition, une étape d'élévation de la température des fibres kératiniques, au moyen d'un moyen de chauffage, à une température comprise entre 110°C et 250°C.

Avantageusement, on utilise un fer comme moyen de chauffage.

Au sens de la présente invention, on entend par « fer » un dispositif de chauffage des fibres kératiniques mettant en contact lesdites fibres et le dispositif de chauffage.

L'extrémité du fer venant en contact avec les cheveux présente généralement deux surfaces planes. Ces deux surfaces planes peuvent être métalliques. Elles peuvent être lisses ou crantées.

A titre d'exemple de fers utilisables dans le procédé selon l'invention, on peut citer tous types de fer plats et, en particulier, de manière non limitative, ceux décrits dans les brevets US 5 957 140, et US 5 046 516.

L'application du fer peut être effectée par touches séparées successives de quelques secondes, ou par déplacement ou glissement progressif le long des mèches.

De préférence, l'application du fer dans le procédé selon l'invention se fait en mouvement continu de la racine à la pointe, en un ou plusieurs passages.

Le procédé selon l'invention peut également comprendre une étape supplémentaire de pré-séchage partiel des fibres kératiniques avant l'étape d'élévation de la température, de manière à éviter d'importants dégagements de vapeurs qui pourraient brûler les mains du coiffeur et le cuir chevelu de la personne. Cette étape de pré-séchage peut se faire par exemple au moyen d'un séchoir, d'un casque, ou bien encore par séchage libre.

L'invention pourra être mieux comprise à l'aide des exemples non limitatifs qui suivent et qui constituent des modes de réalisation préférentiels des compositions selon l'invention.

### EXEMPLE 1:

On réalise une composition de défrisage simplifiée contenant l'urée, à une concentration de 8M dans l'eau, en tant qu'actif de défrisage. Le pH de la composition est de 8,06. On applique cette composition sur des cheveux africains naturellement frisés pendant 15 minutes à une température de 40°C puis on essuie rapidement les cheveux avec une serviette.

On procède ensuite à un lissage mèche à mèche de la chevelure à l'aide d'un fer plat chauffé à 180°C pendant 5 à 10 secondes. Les cheveux sont défrisés efficacement et doux au toucher.

### EXEMPLE 2:

On réalise une composition de défrisage simplifiée contenant l'urée, à une concentration de 4M dans l'eau, en tant qu'actif de défrisage. Le pH de la composition est de 7,7. On applique cette composition sur des cheveux africains naturellement frisés pendant 25 minutes à une température de 40°C puis on essuie rapidement les cheveux avec une serviette.

On procède ensuite à un lissage mèche à mèche de la chevelure à l'aide d'un fer plat chauffé à 180°C pendants 5 à 10 secondes. Les cheveux sont défrisés efficacement et doux au toucher.

## Revendications

1. Procédé de défrisage des cheveux comprenant :
(i) une étape d'application sur les cheveux d'une composition de défrisage contenant au moins un agent dénaturant de masse moléculaire supérieure à 18,1 g/mol et étant une urée de formule (I) 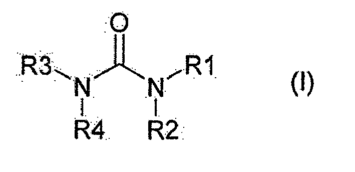 dans laquelle :
R1, R2, R3, R4 représentent, indépendamment : un atome d'hydrogène ou / un radical alkyle ou alcényl inférieur en C1-C4, linéaire ou ramifié, éventuellement substitué par un radical choisi parmi : hydroxyle, amino, diméthylamino, carboxyle ou carboxamide ou N-méthylcarboxamide
- lorsque R1, R2 et R3 représentent un atome d'hydrogène, R4 peut également désigner un radical carboxamide ; méthoxy ; éthoxy ; 1,2,4-triazolyl ; cyclopentyl ; méthoxycarbonyle ; éthoxycarbonyle ; CO-CH=CH-COOH ; phényle éventuellement sustitué par un atome de chlore ou un radical hydroxyle ; benzyle ; ou 2,5-dioxo-4-imidazolidinyle
- lorsque R1 et R3 représentent un atome d'hydrogène, R2 peut également représenter un atome d'hydrogène ou un radical méthyle ou éthyle et R4 un radical acétyle.
- lorsque R1=R2=H, R3 et R4 peuvent également former, avec l'atome d'azote qui les porte, un cycle pipéridine ou 3-méthylpyrazole ou 3,5-diméthylpyrazole ou maléimide.
- R1 et R2 ainsi que R3 et R4 peuvent également former, avec l'atome d'azote qui les porte, un cycle imidazole,
l'agent dénaturant étant présent à une concentration molaire comprise entre 2 M et 8M,
(ii) une étape d'élévation de la température des cheveux, à l'aide d'un moyen de chauffage, à une température comprise entre 110 et 250°C,
après l'étape d'application de la composition de défrisage et avant l'étape d'élévation de la température, on laisse poser la composition pendant 5 à 45 minutes ;
l'agent dénaturant urée de formule (I) étant le seul actif de défrisage.

2. Procédé selon la revendication 1, **caractérisé par le fait qu'**on on élève la température à l'aide du moyen de chauffage à une température comprise entre 120°C et 220°C, plus avantageusement entre 140°C et 220°C.

3. Procédé selon l'une quelconque des revendications précédentes, **caractérisé par le fait que** la masse molaire du dénaturant est comprise entre 40 et 600 g/mol.

4. Procédé selon l'une quelconque des revendications précédentes, **caractérisé par le fait que** la composition est appliquée sur des cheveux humides.

5. Procédé selon l'une quelconque des revendications précédentes, **caractérisé par le fait que** les cheveux sont partiellement préséchés.

6. Procédé selon l'une quelconque des revendications précédentes , **caractérisé par le fait que** le composé de formule (I) est choisi parmi :
- l'urée
- la méthylurée
- l'éthylurée
- la propylurée
- l'isopropylurée
- la n-butylurée
- la sec-butylurée
- l'isobutylurée
- la tert-butylurée
- la cyclopentylurée
- la 1-éthoxyurée
- la 2-hydroxyéthylurée
- la N-(2-hydroxypropyl)urée
- la N-(3-hydroxypropyl)urée
- la N-(2-diméthylaminopropyl)urée
- la N-(3-diméthylaminopropyl)urée
- la 1-(3-hydroxyphényl)urée
- la benzylurée
- le N-carbamoylmaléimide
- le biuret
- l'acide N-carbamoyl maléamique
- le 1-pipéridinecarboxamide
- la 1,2,4-triazol-4-yl-urée
- l'acide hydantoïque
- l'allophanate de méthyle
- l'allophanate d'éthyle
- l'acétylurée
- la 2-hydroxyéthylèneurée
- la 2-(hydroxyéthyl)éthylèneurée
- la N-allyl-N'-éthylurée
- la diallylurée
- la 2-chloroéthylurée
- la N,N-diméthylurée
- la N,N-diéthylurée
- la N,N-dipropylurée
- la 1-cyclopentyl-1-méthylurée
- la 1,3-diméthylurée
- la 1,3-diéthylurée
- la 1,3-bis(2-hydroxyéthyl)urée
- la 1,3-bis(2-hydroxypropyl)urée
- la 1,3-bis(3-hydroxypropyl)urée
- la 1,3-dipropylurée
- la 1-éthyl-3-propylurée
- la 1-sec-butyl-3-méthylylurée
- la 1-isobutyl-3-méthylurée
- la 1-cyclopentyl-3-méthylurée
- la N-acétyl-N'-méthylurée
- la triméthylurée
- la 1-butyl-3,3-diméthylurée
- la tétraméthylurée
- la benzylurée

7. Procédé selon l'une quelconque des revendications précédentes, **caractérisé par le fait que** le composé de formule (I) est choisi parmi :
- l'urée
- la méthylurée
- l'éthylurée
- la propylurée
- la 1-éthoxyurée
- la 2-hydroxyéthylurée
- la N-(2-hydroxypropyl)urée
- la N-(3-hydroxypropyl)urée
- la N-(2-diméthylaminopropyl)urée
- la N-(3-diméthylaminopropyl)urée
- la 1-(3-hydroxyphényl)urée
- le N-carbamoylmaléimide
- l'acide N-carbamoyl maléamique
- le 1-pipéridinecarboxamide
- la 1,2,4-triazol-4-yl-urée
- l'acide hydantoïque
- l'acétylurée
- la 2-hydroxyethylèneurée
- la 2-(hydroxyéthyl)éthylèneurée
- la N-allyl-N'-éthylurée
- la diallylurée
- la 2-chloroéthylurée
- la N,N-diméthylurée
- la 1,3-diméthylurée
- la 1,3-diéthylurée
- la 1,3-bis(2-hydroxyéthyl)urée
- la 1,3-dipropylurée
- la 1-éthyl-3-propylurée
- la N-acétyl-N'-méthylurée
- la benzylurée.

8. Procédé selon l'une quelconque des revendications précédentes, **caractérisé par le fait que** le composé de formule (I) est choisi parmi :
- l'urée
- la méthylurée
- l'éthylurée
- la propylurée

## Patentansprüche

1. Verfahren zum Glätten von Haaren, umfassend:
(i) einen Schritt des Auftragens einer Glättungszusammensetzung auf die Haare, die mindestens ein Denaturierungsmittel mit einem Molekulargewicht von mehr als 18,1 g/mol enthält und bei dem es sich um einen Harnstoff mit der Formel (I) handelt 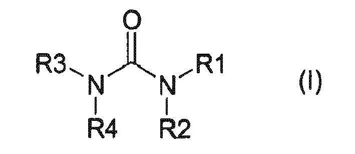 worin:
R1, R2, R3, R4 unabhängig für Folgendes stehen:
ein Wasserstoffatom oder einen linearen oder verzweigten C1-C4-Niederalkyl- oder -Niederalkenylrest, der gegebenenfalls mit einem Rest substituiert ist, ausgewählt aus: Hydroxyl, Amino, Dimethylamino, Carboxyl oder Carboxamid oder N-Methylcarboxamid
- wenn R1, R2 und R3 für ein Wasserstoffatom stehen, kann R4 auch einen Carboxamidrest; Methoxy; Ethoxy; 1,2,4-Triazolyl; Cyclopentyl; Methoxycarbonyl; Ethoxycarbonyl; CO-CH=CH-COOH; Phenyl, das gegebenenfalls mit einem Chloratom oder einem Hydroxylrest substituiert ist; Benzyl; oder 2,5-Dioxo-4-imidazolidinyl bezeichnen,
- wenn R1 und R3 für ein Wasserstoffatom stehen, kann R2 auch für ein Wasserstoffatom oder einen Methyl- oder Ethylrest und R4 für einen Acetylrest stehen,
- wenn R1=R2=H, können R3 und R4 mit dem Stickstoffatom, das sie trägt, auch einen Piperidin- oder 3-Methylpyrazol- oder 3,5-Dimethylpyrazol- oder Maleimidring bilden,
- R1 und R2 sowie R3 und R4 können mit dem Stickstoffatom, das sie trägt, auch einen Imidazolring bilden,
wobei das Denaturierungsmittel in einer Molkonzentration zwischen 2M und 8M vorhanden ist,
(ii) einen Schritt des Erhöhens der Temperatur der Haare mithilfe einer Heizvorrichtung auf eine Temperatur im Bereich zwischen 110 und 250°C,
wobei man die Zusammensetzung nach dem Schritt des Auftragens der Glättungszusammensetzung und vor dem Schritt des Erhöhens der Temperatur für 5 bis 45 Minuten einwirken lässt;
wobei das Harnstoffdenaturierungsmittel mit der Formel (I) der einzige Glättungswirkstoff ist.

2. Verfahren nach Anspruch 1, **dadurch gekennzeichnet, dass** man die Temperatur mithilfe der Heizvorrichtung auf eine Temperatur im Bereich zwischen 120°C und 220°C, stärker bevorzugt zwischen 140°C und 220°C erhöht.

3. Verfahren nach einem der vorhergehenden Ansprüche, **dadurch gekennzeichnet, dass** das Molekulargewicht des Denaturierungsmittels im Bereich zwischen 40 und 600 g/mol liegt.

4. Verfahren nach einem der vorhergehenden Ansprüche, **dadurch gekennzeichnet, dass** die Zusammensetzung auf feuchte Haare aufgetragen wird.

5. Verfahren nach einem der vorhergehenden Ansprüche, **dadurch gekennzeichnet, dass** die Haare teilweise vorgetrocknet sind.

6. Verfahren nach einem der vorhergehenden Ansprüche, **dadurch gekennzeichnet, dass** die Verbindung mit der Formel (I) ausgewählt ist aus:
- Harnstoff
- Methylharnstoff
- Ethylharnstoff
- Propylharnstoff
- Isopropylharnstoff
- n-Butylharnstoff
- sec-Butylharnstoff
- Isobutylharnstoff
- tert-Butylharnstoff
- Cyclopentylharnstoff
- 1-Ethoxyharnstoff
- 2-Hydroxyethylharnstoff
- N-(2-Hydroxypropyl)harnstoff
- N-(3-Hydroxypropyl)harnstoff
- N-(2-Dimethylaminopropyl)harnstoff
- N-(3-Dimethylaminopropyl)harnstoff
- 1-(3-Hydroxyphenyl)harnstoff
- Benzylharnstoff
- N-Carbamoylmaleimid
- Biuret
- N-Carbamoylmaleinamidsäure
- 1-Piperidincarboxamid
- 1,2,4-Triazol-4-yl-harnstoff
- Hydantoinsäure
- Methylallophanat
- Ethylallophanat
- Acetylharnstoff
- 2-Hydroxyethylenharnstoff
- 2-(Hydroxyethyl)ethylenharnstoff
- N-Allyl-N'-ethylharnstoff
- Diallylharnstoff
- 2-Chlorethylharnstoff
- N,N-Dimethylharnstoff
- N,N-Diethylharnstoff
- N,N-Dipropylharnstoff
- 1-Cyclopentyl-1-methylharnstoff
- 1,3-Dimethylharnstoff
- 1,3-Diethylharnstoff
- 1,3-bis(2-Hydroxyethyl)harnstoff
- 1,3-bis(2-Hydroxypropyl)harnstoff
- 1,3-bis(3-Hydroxypropyl)harnstoff
- 1,3-Dipropylharnstoff
- 1-Ethyl-3-propylharnstoff
- 1-sec-Butyl-3-methylylharnstoff
- 1-Isobutyl-3-methylharnstoff
- 1-Cyclopentyl-3-methylharnstoff
- N-Acetyl-N'-methylharnstoff
- Trimethylharnstoff
- 1-Butyl-3,3-dimethylharnstoff
- Tetramethylharnstoff
- Benzylharnstoff.

7. Verfahren nach einem der vorhergehenden Ansprüche, **dadurch gekennzeichnet, dass** die Verbindung mit der Formel (I) ausgewählt ist aus:
- Harnstoff
- Methylharnstoff
- Ethylharnstoff
- Propylharnstoff
- 1-Ethoxyharnstoff
- 2-Hydroxyethylharnstoff
- N-(2-Hydroxypropyl)harnstoff
- N-(3-Hydroxypropyl)harnstoff
- N-(2-Dimethylaminopropyl)harnstoff
- N-(3-Dimethylaminopropyl)harnstoff
- 1-(3-Hydroxyphenyl)harnstoff
- N-Carbamoylmaleimid
- N-Carbamoylmaleinamidsäure
- 1-Piperidincarboxamid
- 1,2,4-Triazol-4-yl-harnstoff
- Hydantoinsäure
- Acetylharnstoff
- 2-Hydroxyethylenharnstoff
- 2-(Hydroxyethyl)ethylenharnstoff
- N-Allyl-N'-ethylharnstoff
- Diallylharnstoff
- 2-Chlorethylharnstoff
- N,N-Dimethylharnstoff
- 1,3-Dimethylharnstoff
- 1,3-Diethylharnstoff
- 1,3-bis(2-Hydroxyethyl)harnstoff
- 1,3-Dipropylharnstoff
- 1-Ethyl-3-propylharnstoff
- N-Acetyl-N'-methylharnstoff
- Benzylharnstoff.

8. Verfahren nach einem der vorhergehenden Ansprüche, **dadurch gekennzeichnet, dass** die Verbindung mit der Formel (I) ausgewählt ist aus:
- Harnstoff
- Methylharnstoff
- Ethylharnstoff
- Propylharnstoff.

## Claims

1. Process for relaxing keratin fibres comprising:
(i) a step of applying to the hair a relaxing composition containing at least one denaturing agent with a molecular mass of greater than 18.1 g/mol, and being an urea of formula (I): 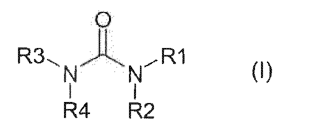 in which:
R1, R2, R3 and R4 represent, independently:
a hydrogen atom, or
a linear or branched lower C1-C4 alkyl or alkenyl radical, optionally substituted with a radical chosen from: hydroxyl, amino, dimethylamino, carboxyl or carboxamide or N-methylcarboxamide
- when R1, R2 and R3 represent a hydrogen atom, R4 may also denote a radical chosen from the following: carboxamide; methoxy; ethoxy; 1,2,4-triazolyl; cyclopentyl; methoxycarbonyl; ethoxycarbonyl; CO-CH=CH-COOH; phenyl optionally substituted with a chlorine atom or a hydroxyl radical; benzyl; or 2,5-dioxo-4-imidazolidinyl
- when R1 and R3 represent a hydrogen atom, R2 may also represent a hydrogen atom or a methyl or ethyl radical and R4 an acetyl radical
- when R1=R2=H, R3 and R4 may also form, with the nitrogen atom that bears them, a piperidine or 3-methylpyrazole or 3,5-dimethylpyrazole or maleimide ring
- R1 and R2, and also R3 and R4, may also form, with the nitrogen atom that bears them, an imidazole ring, the denaturing agent being present in a molar concentration of between 2M and 8M,
(ii) a step of raising the temperature of the hair, using a heating means, to a temperature of between 110 and 250°C,
after the step of applying the relaxing composition and before the step of raising the temperature, the composition is left to stand for 5 to 45 minutes;
the urea denaturing agent of formula (I) being the sole relaxing active agent.

2. Process according to Claim 1,
**characterized in that** the temperature is raised using a means of heating to a temperature of between 120°C and 220°C and more advantageously between 140°C and 220°C.

3. Process according to either of the preceding claims, **characterized in that** the molar mass of the denaturing agent is between 40 and 600 g/mol.

4. Process according to any one of the preceding claims, **characterized in that** the composition is applied to wet hair.

5. Process according to any one of the preceding claims, **characterized in that** the hair is partially predried.

6. Process according to any one of the preceding claims, **characterized in that** the compound of formula (I) is chosen from:
- urea
- methylurea
- ethylurea
- propylurea
- isopropylurea
- n-butylurea
- sec-butylurea
- isobutylurea
- tert-butylurea
- cyclopentylurea
- 1-ethoxyurea
- 2-hydroxyethylurea
- N-(2-hydroxypropyl)urea
- N-(3-hydroxypropyl)urea
- N-(2-dimethylaminopropyl)urea
- N-(3-dimethylaminopropyl)urea
- 1-(3-hydroxyphenyl)urea
- benzylurea
- N-carbamoylmaleimide
- biuret
- N-carbamoylmaleamic acid
- 1-piperidinecarboxamide
- 1,2,4-triazol-4-ylurea
- hydantoic acid
- methyl allophanate
- ethyl allophanate
- acetylurea
- 2-hydroxyethyleneurea
- 2-(hydroxyethyl)ethyleneurea
- N-allyl-N'-ethylurea
- diallylurea
- 2-chloroethylurea
- N,N-dimethylurea
- N,N-diethylurea
- N,N-dipropylurea
- 1-cyclopentyl-1-methylurea
- 1,3-dimethylurea
- 1,3-diethylurea
- 1,3-bis(2-hydroxyethyl)urea
- 1,3-bis(2-hydroxypropyl)urea
- 1,3-bis(3-hydroxypropyl)urea
- 1,3-dipropylurea
- 1-ethyl-3-propylurea
- 1-sec-butyl-3-methylurea
- 1-isobutyl-3-methylurea
- 1-cyclopentyl-3-methylurea
- N-acetyl-N'-methylurea
- trimethylurea
- 1-butyl-3,3-dimethylurea
- tetramethylurea
- benzylurea.

7. Process according to any one of the preceding claims, **characterized in that** the compound of formula (I) is chosen from:
- urea
- methylurea
- ethylurea
- propylurea
- 1-ethoxyurea
- 2-hydroxyethylurea
- N-(2-hydroxypropyl)urea
- N-(3-hydroxypropyl)urea
- N-(2-dimethylaminopropyl)urea
- N-(3-dimethylaminopropyl)urea
- 1-(3-hydroxyphenyl)urea
- N-carbamoylmaleimide
- N-carbamoylmaleamic acid
- 1-piperidinecarboxamide
- 1,2,4-triazol-4-ylurea
- hydantoic acid
- acetylurea
- 2-hydroxyethyleneurea
- 2-(hydroxyethyl)ethyleneurea
- N-allyl-N'-ethylurea
- diallylurea
- 2-chloroethylurea
- N,N-dimethylurea
- 1,3-dimethylurea
- 1,3-diethylurea
- 1,3-bis(2-hydroxyethyl)urea
- 1,3-dipropylurea
- 1-ethyl-3-propylurea
- N-acetyl-N'-methylurea
- benzylurea.

8. Process according to any one of the preceding claims, **characterized in that** the compound of formula (I) is chosen from:
- urea
- methylurea
- ethylurea
- propylurea.
